# EUROPEAN PATENT APPLICATION

(11) **EP 4 559 427 A2**
(43) Date of publication of application: **28.05.2025**
(21) Application number: 24214469.9
(22) Date of filing: 21.11.2024
(51) Int. Cl.: A61B 50/10, A61B 50/20

(54) **DEVICE FOR HOLDING A MEDICAL INSTRUMENT, IN PARTICULAR AN ENDOSCOPE**

(30) Priority: 22.11.2023 DE 102023132550
(71) Applicant: Olympus Winter & Ibe GmbH, 22045 Hamburg (DE)
(72) Inventor: Ouweltjes, Koen, 1019 PZ Amsterdam (NL); Ringelberg, Kornel, 1276 MK Huizen (NL)
(74) Representative: Eisenführ Speiser

(57) **Abstract**

The invention relates to a holding device (1) for holding a medical instrument, in particular an endoscope. Further the invention relates to a cabinet (90) as well as to a method (700) for holding a medical instrument and a use of a holding device (1). The holding device (1) comprises: a fixed element (10) that extends from a first end (11) to a second end (12), a holding arm (20) that extends from a first end (21) to a second end (22), wherein the first end (21) of the holding arm (20) is rotatably connected to the second end (12) of the fixed element (10) in such a way that the holding arm (20) is rotatable about a holding arm axis (200) relative to the fixed element (10) and movable by rotating the holding arm (20) about the holding arm axis (200) between a storage position and a loading position, and a receiving element (40) for receiving a medical instrument, in particular an endoscope, wherein the receiving element (40) is rotatably connected to the second end (22) of the holding arm (20) in such a way that the receiving element (40) is rotatable about a receiving element axis (400) relative to the holding arm (20).

## Description

The invention relates to a holding device for holding a medical instrument, in particular an endoscope, and a cabinet for storing and/or drying a medical instrument. Further, the invention relates to a method for holding a medical instrument and a use of a holding device.

Reprocessing of medical instruments, in particular of flexible endoscopes, requires adequate drying and storage after high-level disinfection. Adequate storing and drying of medical instruments such as endoscopes is crucial for preventing infections of patients and has become more and more important for preventing infections of patients.

Different types of drying and/or storage cabinets for endoscopes exists. In particular, cabinets in which endoscopes are arranged vertically and cabinets in which endoscopes are arranged horizontal are known.

Storing endoscopes in a vertical position has the advantage that no kinking of endoscope channels occurs so that typically adequate drying of the endoscopes can be achieved in this position. However, known storage cabinets in which endoscopes are arranged vertically typically have a bad operator convenience. Often, holding elements that hold the endoscopes are difficult to reach, because the holding elements are arranged at a relatively high height in the storage cabinet. This makes loading and unloading of endoscopes relatively difficult and/or unpleasant.

Therefore, it is an object of the present invention to provide an improved solution for holding and/or storing a medical instrument, in particular an endoscope, in a storage cabinet. In particular it is an object of the present invention to provide a solution that provides a reliable storage and reliable drying of the medical instruments, in particular endoscopes, and at the same time an improved operator convenience.

According to a first aspect, it is provided a holding device for holding a medical instrument, in particular an endoscope. The holding device comprises a fixed element that extends from a first end of the fixed element to a second end of the fixed element. The holding device further comprises a holding arm that extends from a first end of the holding arm to a second end of the holding arm, wherein the first end of the holding arm is rotatably connected to the second end of the fixed element in such a way that the holding arm is rotatable about a holding arm axis relative to the fixed element and movable by rotating the holding arm about the holding arm axis between a storage position and a loading position. The holding device further comprises a receiving element for receiving a medical instrument, in particular an endoscope, wherein the receiving element is rotatably connected to the second end of the holding arm in such a way that the receiving element is rotatable about a receiving element axis relative to the holding arm.

Preferably the fixed element is configured to be connected to a cabinet for storing and/or drying a medical instrument, in particular an endoscope, in particular to the inner and/or lower side of a ceiling element of such a cabinet, so that the fixed element extends inside the cabinet along a vertical axis.

Preferably the holding arm is rotatably mounted on the fixed element so that it is rotatable about the holding arm axis. The holding arm axis is preferably arranged horizontally.

The storage position is in particular to be understood as a position in which a medical device, in particular an endoscope, is hold by the holding element and stored in the cabinet in which the holding element is arranged. In the storage position, the holding arm is preferably arranged vertically. In the storage position, preferably the holding arm and the fixed element are arranged parallel to each other.

The loading position is in particular to be understood as a position for arranging a medical device, in particular an endoscope, onto the holding device and for removing a medical device, in particular an endoscope, from the holding device. In the loading position, the holding arm is preferably not arranged vertically.

The receiving element is preferably configured to hold a medical instrument. A medical instrument can in particular be arranged onto the receiving element in such a way that it is placed directly onto the receiving element and in contact with the receiving element.

Preferably the receiving element is rotatably mounted on the holding arm so that it is rotatable about the receiving element axis. The receiving element axis is preferably arranged horizontally. The receiving element axis is preferably oriented parallel and distanced to the holding arm axis.

An advantage of such a holding element is that due to the rotatable holding arm and the rotatable receiving element, it is possible to rotate the holding arm about the holding arm axis and the receiving element about the receiving element axis by the same angle so that the receiving element can be kept in a position in which it is not rotated in space when the holding arm is moved between the storage position and the loading position.

This leads to the advantage that the medical instrument can be placed onto the receiving element in the loading position in a vertically extending position and be moved from the loading position to the storage position while keeping its vertically extending orientation during the movement and also when arranged in the storage position.

Another advantage is that the receiving element is in a lower position when compared to the storage position so that the medical instrument can be placed onto the receiving element much easier. Furthermore, the receiving element is rotated towards the user so that placing a medical device onto the receiving element becomes easier for the user.

It is particularly preferred that the holding arm has a longitudinal extension between the first end of the holding arm and the second end of the holding arm, and wherein the holding arm is arranged in such a way that in the storage position the longitudinal extension of the holding arm is substantially vertical and the second end of the holding arm lies vertically above the first end of the holding arm, and/or in the loading position the longitudinal extension of the holding arm is not substantially vertical.

Arranging the holding arm substantially vertically in the storage position has the advantage that the holding device has a compact shape in the storage position and relatively little space is needed for the holding device and for storing the medical instrument. This way it is possible to store multiple medical instruments on multiple holding devices within a relatively small space.

Another advantage of arranging the holding arm substantially vertically in the storage position is that this way the medical instrument can be placed in a relatively high position and when rotating the holding arm to the loading position the medical instrument can be placed much lower than in the storage position.

Preferably in the storage position the second end of the holding arm is arranged in an upper position and in the loading position the second end of the holding arm is arranged in a lower position.

It is particularly preferred that the fixed element has a longitudinal extension between the first end of the fixed element and the second end of the fixed element, wherein the longitudinal extension of the fixed element is substantially vertical and the first end of the fixed element lies vertically above the second end of the fixed element.

It is particularly preferred that the receiving element comprises a first support part with a first support surface that has a longitudinal extension of the receiving element, wherein the longitudinal extension of the receiving element is substantially vertical when the holding arm is arranged in the storage position and substantially vertical when the holding arm is arranged in the loading position, and preferably substantially vertical when the holding arm is arranged in any position between the storage position and the loading position.

Preferably the longitudinal extension of the receiving element is arranged substantially vertical in any possible position of the receiving element.

Such a first support surface can be particularly advantageous to prevent rotation of a medical instrument when it is arranged on the receiving element.

It is particularly preferred that the receiving element comprises a second support part with a second support surface that extends in a substantially horizontal direction when the holding arm is arranged in the storage position and that extends in a substantially horizontal direction when the holding arm is arranged in the loading position, and that preferably extends substantially horizontal when the holding arm is arranged in any position between the storage position and the loading position.

Preferably the second support surface extends substantially horizontal in any possible position of the receiving element.

Such a second support surface can be particularly advantageous to prevent rotation of a medical instrument when it is arranged on the receiving element.

It is particularly preferred that the holding arm axis and the receiving element axis are arranged parallel to each other and preferably horizontally.

It is particularly preferred that in the loading position of the holding element the holding arm axis is arranged in a vertically higher position than the receiving element axis. This can in particular be achieved when the holding element is rotated about the holding arm axis about an angle of above 90°. An advantage of such an arrangement is that the receiving element can then be easily reached for arranging a medical instrument onto the receiving element or for removing a medical instrument from the receiving element.

It is particularly preferred that in the storage position of the holding element the holding arm axis is arranged in a vertically lower position than the receiving element axis. Preferably in the storage position of the holding element the holding arm axis lies exactly vertically below the receiving element axis.

It is particularly preferred that the holding arm is rotatable about the holding arm axis through a first angle of rotation between the storage position and the loading position, wherein the receiving element is rotatable about the receiving element axis through a second angle of rotation between the storage position and the loading position, and wherein the first angle of rotation and the second angle of rotation are substantially equal in amount.

An advantage of such angles of rotation is that this way it is possible to keep the receiving element in a position in which it is not rotated in space when the holding arm is rotated between the storage position and the loading position.

Preferably the first angle of rotation and the second angle of rotation are each larger than 90°. Preferably the first angle of rotation is an angle that is larger than 90°, in particular larger than 100°, and preferably smaller than 180°, in particular smaller than 160°. Preferably the second angle of rotation is an angle that is largerthan 90°, in particular larger than 100°, and preferably smaller than 180°, in particular smaller than 160°.

It is particularly preferred that the first end of the holding arm is rotatably connected to the second end of the fixed element by means of a first connecting device, and wherein the receiving element is rotatably connected to the second end of the holding arm by means of a second connecting device.

The first connecting device is preferably configured to connect the holding arm and the fixed element. The second connecting device is preferably configured to connect the receiving element and the holding arm.

It is particularly preferred that the first connecting device is configured and arranged to rotate the holding arm about the holding arm axis through a first angle of rotation between the storage position and the loading position, wherein the second connecting device is configured and arranged to rotate the receiving element about the receiving element axis through a second angle of rotation between the storage position and the loading position, and wherein the first angle of rotation and the second angle of rotation are substantially equal in amount.

An advantage of providing connecting elements that are configured to rotate the holding arm and the receiving element is that this way it is possible to keep the receiving element in a position in which it is not rotated in space when the holding arm is rotated between the storage position and the loading position.

Preferably the first angle of rotation and the second angle of rotation are each larger than 90°. Preferably the first angle of rotation is an angle that is larger than 90°, in particular larger than 100°, and preferably smaller than 180°, in particular smaller than 160°. Preferably the second angle of rotation is an angle that is largerthan 90°, in particular larger than 100°, and preferably smaller than 180°, in particular smaller than 160°.

It is particularly preferred that the first connecting device and the second connecting device are coupled to each other in such a way that a rotation of the holding arm about the holding arm axis causes a rotation of the receiving element about the receiving element axis that is substantially equal in amount.

By coupling the first connecting element and the second connecting element to each other in this way, it can be achieved that the angle about which the holding arm and the receiving element rotate are substantially equal so that a medical device placed on the receiving element is not rotated about its own axis when the holding arm moves between the storage position and the loading position.

It is particularly preferred that the first connecting device and the second connecting device are coupled to each other by means of a belt, in particular a toothed belt, that is connected to the first connecting device and to the second connecting device.

With such a belt it can be achieved that the first connecting device and the second connecting device are coupled in a reliable way because a rotation of the receiving element about the receiving element axis then inevitably causes a rotation of the holding arm about the holding arm axis, and vice versa. Another advantage of such a belt is that the angle about which the holding arm is rotated and the angle about which the receiving element is rotated are in any position of the holding element and the receiving element equal in amount, yet in opposite direction. If a toothed belt is used slippage can be avoided even more reliably.

Preferably the belt is enclosed by a housing of the holding arm. By enclosing the belt in a housing, the belt can be protected and the used does not get in direct contact with the coupling mechanism.

It is particularly preferred that the holding arm comprises a tensioning wheel that is in contact with the belt and configured and arranged to tension the belt. Preferably, the tensioning wheel is movable relative to the belt for tensioning the belt. Preferably the tensioning wheel can be moved by using a screw driver. With such a tensioning wheel the belt can be tensioned to provide a tensioning that is optimal to transfer the rotation between the first connecting device and the second connecting device.

It is particularly preferred that the first connecting device comprises a rotary damper that is connected to the second end of the fixed element, in particular via a rotary damper holder that is connected to the second end of the fixed element, wherein the rotary damper comprises at least one rotary damping gear, preferably two rotary damping gears

A rotary damper is in particular to be understood to be a mechanical component that is configured to slow down a movement through continuous rotation by means of a damping torque and thus to allow controlled and comfortable use of the holding element.

With such a rotary damper the handling of the holding device can be improved, because the movement of the holding arm, and thus also of the receiving element, are smooth and therefore better controllable.

Preferably the at least one rotary damping gear meshes with a first connecting device gear that is connected to the first end of the holding arm in a rotationally fixed manner. Preferably two rotary damping gears mesh with the first connecting device gear.

It is particularly preferred that the at least one damping gear is configured and arranged to provide a damping torque against a rotational movement of the holding arm about the holding arm axis for slowing down a rotation of the holding arm about the holding arm axis.

It is particularly preferred that the holding device comprises an engaging mechanism, preferably comprising a spring element, in particular formed as a spring plate, and/or at least one latching element, wherein the engaging mechanism is configured and arranged to provide a locking force to the holding arm and/or to the receiving element when the holding arm is arranged in the storage position and/or to provide a locking force to the holding arm and/orto the receiving element when the holding arm is arranged in the loading position.

An advantage of such an engaging mechanism is that it allows for giving the user of the holding device a haptic and/or acoustic feedback when the correct storage position and/or the correct loading position of the holding arm is reached. This feedback can be produced by the spring element that can preferably lock into a locking position when the holding arm is in the storage position and/or in the loading position.

Preferably, in the storage position and/or in the storage position a locking force acts on the spring element so that the user has to overcome the locking force, which should be possible by hand and without using excessive force, in order to move the holding arm away from the storage position and/or the loading position.

Preferably, no locking force acts on the spring element when the holding arm is arranged in a position different than the storage position and/or the loading position.

Another advantage of such an engaging mechanism is that it allows for securing the holding arm in the storage position so that the holding arm is securely kept in the storage position until the locking force is overcome by a user pulling the holding element away from the storage position. This way the medical instrument that shall be stored on the receiving element is securely and reliably kept in the storage position until a user changes the position.

It is particularly preferred that the holding device comprises a state indicator device that is configured to indicate a state of the holding device and/or a state of a medical instrument that is arranged on the receiving element, wherein preferably the state indicator device is arranged on the fixed element.

A state indicator device is in particular understood to be a status indicator device. The state indicator device is preferably configured to display a state, which means a status of the holding device and/or of the medical instrument that is arranged on the receiving element. Here and in the following the terms state and status can be used interchangeably.

Preferably the state indicator device is at least partly integrated in the fixed element, in particular at the second end of the fixed element and/or at a rotary damper holder that is connected to the second end of the fixed element.

Preferably the state indicator device comprises at least one light-emitting diode. Preferably the state indicator device comprises a slit and a light-emitting diode that is arranged inside and/or behind the slit, wherein the slit preferably extends along a vertical direction. Preferably the at least one light-emitting diode is positioned on the side of the holding arm that faces the door of a cabin when the holding device is arranged inside the cabin and connected to the cabin in its operating position.

Preferably the state indicator device is configured to display different states of the holding device and/or of a medical instrument that is arranged on the receiving element by means of at least one state light that is configured to show different colors for different states of the holding device and/or of a medical instrument that is arranged on the receiving element. Preferably the different states are displayed dependent on sensor data that is obtained by at least one sensor and/or dependent on the duration of a medical instrument being arranged on the receiving element. Preferably the different states comprise at least one, in particular all, of the following states: the holding device is free to use and/or no medical instrument is arranged on the receiving element; a medical instrument that is arranged on the receiving element is being dried; a medical instrument that is arranged on the receiving element is dry; a problem is detected.

Preferably the state indicator device indicates each state by displaying different colors that are each assigned to one of the states.

According to a further aspect, it is provided a cabinet for storing and/or drying a medical instrument, in particular an endoscope, the cabinet comprising: at least one holding device, preferably several holding devices, as described herein, wherein the at least one holding element is arranged inside of the cabinet.

Preferably the cabinet is configured to store a medical instrument, in particular an endoscope. The cabinet preferably comprises sidewalls, a back wall, a ceiling element, and a front door, and preferably a bottom wall, wherein these elements enclose a storage room that is accessible and closable via the front door. The front door can be opened, in particular for loading a medical instrument to the at least one holding device, and closed, in particular to securely store the medical instrument within the storage room of the cabinet.

Preferably multiple, for example six, holding elements are arranged inside of the cabinet. Preferably the holding elements are arranged in a row next to each other, wherein each of the holding elements is connected to a ceiling element of the cabinet via its fixed element.

Preferably the holding arm is movable from the storage position to the loading position in such a way that the holding arm is rotated into the direction of the door of the cabinet. This allows for an improved usability, as the holding arm, and thus the receiving element, is moved towards the user to the loading position so that the user can reach the receiving element and the holding arm particularly easily.

The cabinet is preferably connected to a drying gas supply so that the holding devices can be supplied with drying gas to dry the medical instruments when they are arranged on the receiving elements of the holding devices. Preferably a drying gas supply line extends at least partly through the fixed element of the holding device. Preferably the holding device comprises a drying gas outlet, preferably arranged on the fixed element of the holding device, wherein the drying gas outlet can be connected to the medical instrument when the medical instrument is arranged on the receiving element. This way drying gas can be provided to dry the medical instrument in a particularly preferred manner.

Preferably the fixed element of the at least one holding device is mounted to a ceiling element of the cabinet. This way, the holding device can be arranged at an upper position within a cabinet.

Preferably at least one sensor for obtaining sensor data is arranged in the upper part of the cabinet, in particular on the inner side of the ceiling element of the cabinet.

Preferably multiple sensors for obtaining sensor data are arranged in the upper part of the cabinet, in particular on the inner side of the ceiling element of the cabinet. Preferably for each holding device a sensor is provided. Preferably vertically above each receiving element a sensor is arranged on the ceiling element of the cabinet.

Preferably the at least one sensor is configured to obtain sensor data that contain one or more, preferably all, of the following information: the holding device is free to use and/or no medical instrument is arranged on the receiving element; a medical instrument that is arranged on the receiving element is being dried; a medical instrument that is arranged on the receiving element is dry; a problem is detected. Preferably a drying duration can be determined by using sensor data obtained by the at least one sensor.

Preferably at least one control device is provided, and preferably arranged in the cabinet, wherein the at least one control device is signally connected to the at least one sensor and to the state indicator device. The at least one control device is configured to receive sensor date from the at least one sensor and to provide the state indicator device with information on a state of the holding device and/or of a medical instrument that is arranged on the receiving element. Alternatively the state indicator device can be directly signally connected to the at least one sensor to receive information on a state of the holding device and/or of a medical instrument that is arranged on the receiving element.

Preferably the different states are displayed by the state indicator device dependent on sensor data that is obtained by at least one sensor and/or dependent on the duration of a medical instrument being arranged on the receiving element. Preferably the different states comprise at least one, in particular all, of the following states: the holding device is free to use and/or no medical instrument is arranged on the receiving element; a medical instrument that is arranged on the receiving element is being dried; a medical instrument that is arranged on the receiving element is dry; a problem is detected.

According to a further aspect, it is provided a method for holding a medical instrument, in particular an endoscope, the method comprising: providing a holding device, preferably a holding device as described herein, wherein the holding device comprises: a fixed element, a holding arm, wherein the holding arm is rotatably connected to the fixed element in such a way that the holding arm is rotatable about a holding arm axis relative to the fixed element, and a receiving element for receiving a medical instrument, in particular an endoscope, wherein the receiving element is rotatably connected to the holding arm in such a way that the receiving element is rotatable about a receiving element axis relative to the holding arm, moving the holding arm from a storage position, in which the holding arm is arranged substantially vertically, to a loading position by rotating the holding arm about the holding arm axis through a first angle of rotation, whereby the receiving element is rotated about the receiving element axis through a second angle of rotation, wherein preferably the first angle of rotation and the second angle of rotation are substantially equal in amount.

It is particularly preferred that the method comprises: placing a medical instrument, in particular an endoscope, onto the receiving element, and rotating the holding arm about the holding arm axis back into the storage position.

Preferably when the storage position is reached a haptic and/or acoustic feedback is generated by an engaging mechanism, preferably comprising a spring element and/or at least one latching element.

Preferably the method comprises: giving the user of the holding device a haptic and/or acoustic feedback when the correct storage position and/or loading position of the holding arm is reached. This feedback can in particular be produced by the spring element that preferably locks into a locking position when the holding arm is in the storage position and/or in the loading position. Preferably, in the storage position and/or in the loading position a locking force acts on the spring element. The method preferably comprises that the user overcomes the locking force by hand and without using excessive force to move the holding arm away from the storage position towards the loading position. Preferably, no locking force acts on the spring element when the holding arm is arranged in a position different than the storage position and/or the loading position.

According to a further aspect, it is provided a use of a holding device as described herein for holding an endoscope, in particular in a cabinet as described herein.

As to the advantages, preferred embodiments and details of the individual different aspects and their preferred embodiments, reference is also made to the corresponding advantages, preferred embodiments and details described with reference to the respective other aspects.

Further advantageous embodiments result from the combination of individual, several or all of the preferred features described herein. Preferred embodiments shall now be described with reference to the attached drawings, in which
- Fig. 1a:: a side view of a holding device, wherein the holding arm is arranged in the storage position;
- Fig. 1b:: a side view of a holding device, wherein the holding arm is arranged in the loading position;
- Fig. 2:: a perspective view of a holding device, wherein the holding arm is arranged in the storage position;
- Fig. 3:: an exploded view of the connection of the first end of the holding arm with the second end of the fixed element by means of a first connecting device;
- Fig. 4:: a perspective view of the inner part of the holding arm;
- Fig. 5:: a perspective view of a holding device, wherein the holding arm is arranged in the storage position and the receiving element is removed;
- Fig. 6:: an exploded view of the engaging mechanism;
- Fig. 7:: a schematic view of a cabinet;
- Fig. 8:: a schematic view of a method for holding a medical instrument.

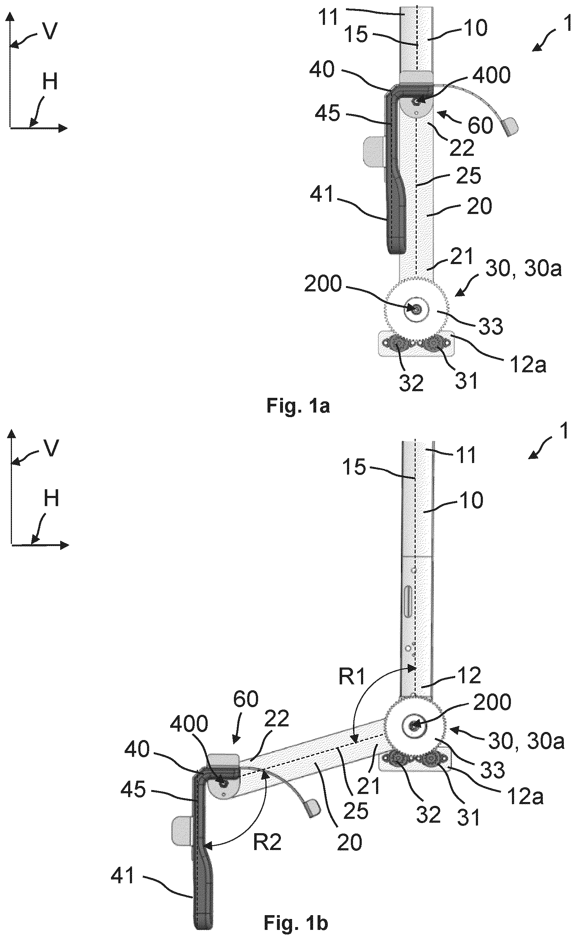

Fig. 1a and Fig. 1b each show a side view of the same holding device 1, wherein in Fig. 1a a storage position is shown and in Fig. 1b a loading position is shown. The holding device 1 is configured to hold a medical instrument (not shown), in particular an endoscope. The holding device 1 comprises a fixed element 10 that extends from a first end 11 of the fixed element 10 to a second end 12 of the fixed element 10. The holding device 1 further comprises a holding arm 20 that extends from a first end 21 of the holding arm 20 to a second end 22 of the holding arm 20. The first end 21 of the holding arm 20 is rotatably connected to the second end 12 of the fixed element 10 in such a way that the holding arm 20 is rotatable about a holding arm axis 200 relative to the fixed element 10 and movable by rotating the holding arm 20 about the holding arm axis 200 between the storage position that is shown in Fig. 1a and the loading position that is shown in Fig. 1b. The holding device 1 comprises a receiving element 40 for receiving a medical instrument, in particular an endoscope, wherein the receiving element 40 is rotatably connected to the second end 22 of the holding arm 20 in such a way that the receiving element 40 is rotatable about a receiving element axis 400 relative to the holding arm 20.

The fixed element 10 has a longitudinal extension 15 between the first end 11 of the fixed element 10 and the second end 12 of the fixed element 10. The longitudinal extension 15 of the fixed element 10 is substantially vertical and the first end 11 of the fixed element 10 lies vertically above the second end 12 of the fixed element 10.

The holding arm 20 has a longitudinal extension 25 between its first end 21 and its second end 22. The holding arm 20 is arranged in such a way that in the storage position the longitudinal extension 25 of the holding arm 20 is arranged substantially in a vertical direction V and the second end 22 of the holding arm 20 lies vertically above the first end 21 of the holding arm 20, as shown in Fig. 1a. In the loading position the longitudinal extension 25 of the holding arm 20 is not arranged in a vertical direction V and the second end 22 of the holding arm 20 lies vertically below the first end 21 of the holding arm 20, as shown in Fig. 1b.

The holding arm 20 is rotatable about the holding arm axis 200 through a first angle of rotation R1 between the storage position (as shown in Fig. 1a) and the loading position (as shown in Fig. 1b). The receiving element 40 is rotatable about the receiving element axis 400 through a second angle of rotation R2 between the storage position (as shown in Fig. 1a) and the loading position (as shown in Fig. 1b). The first angle of rotation R1 and the second angle of rotation R2 are equal in amount. The first angle of rotation R1 and the second angle of rotation R2 can for example be in the range of about 100° to 120°.

The first end 21 of the holding arm 20 is rotatably connected to the second end 12 of the fixed element 10 via a rotary damper holder 12a that is connected to or part of the second end 12 of the fixed element 10 by means of a first connecting device 30 that comprises a rotary damper 30a that is connected to the second end 12 of the fixed element 10, wherein the rotary damper 30a comprises two rotary damping gears 31, 32 that mesh with a first connecting device gear 33.

The receiving element 40 comprises a first support part 41 with a longitudinal extension 45, wherein the longitudinal extension 45 is arranged substantially in a vertical direction V when the holding arm 20 is arranged in the storage position, as shown in Fig. 1a. The longitudinal extension 45 is also arranged substantially in a vertical direction V when the holding arm 20 is arranged in the loading position, as shown in Fig. 1b. The longitudinal extension 45 is further arranged substantially in a vertical direction V in any position between the storage position and the loading position. The receiving element 40 is rotatably connected to the second end 22 of the holding arm 20 by means of a second connecting device 60.

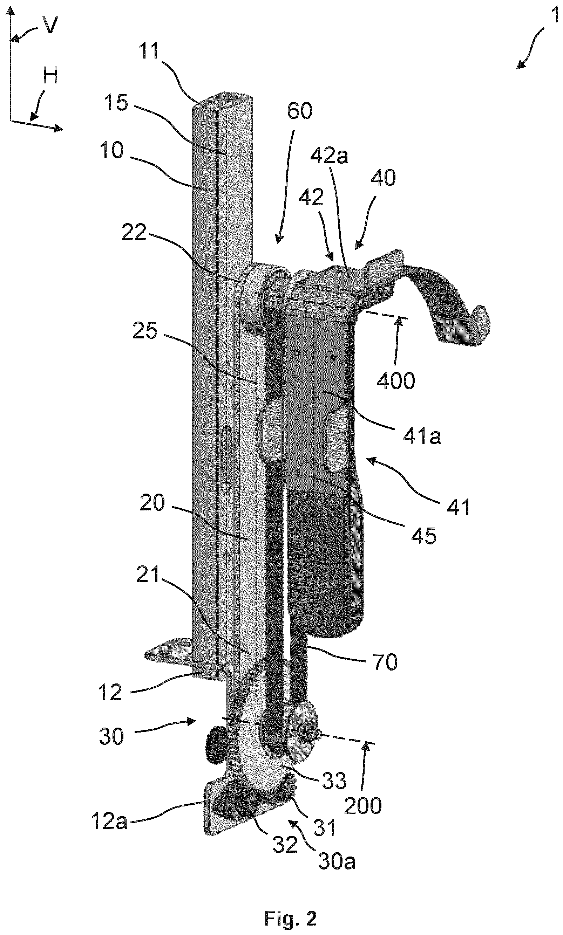

Fig. 2 shows a perspective view of a holding device 1, wherein the holding arm 20 (from which the cover is not shown here) is arranged in the storage position. The holding device 1 shown in Fig. 2 is designed as the holding device shown in Fig. 1a and Fig. 1b. Therefore, it is referred to the description of Fig. 1a and Fig. 1b above. However, some features are shown in Fig. 2 that are not visible in Fig. 1a and Fig. 1b and that are described in the following.

The receiving element 40 comprises a first support part 41 with a first support surface 41a that has a longitudinal extension 45 of the receiving element 40, wherein the longitudinal extension 45 of the receiving element 40 is substantially vertical (i.e. oriented parallel to the vertical direction V) when the holding arm 20 is arranged in the storage position. It is also substantially vertical when the holding arm 20 is arranged in the loading position, and furthermore substantially vertical when the holding arm 20 is arranged in any position between the storage position and the loading position.

The receiving element 40 comprises a second support part 42 with a second support surface 42a that extends in a substantially horizontal direction (i.e. it is oriented parallel to the horizontal direction H) when the holding arm 20 is arranged in the storage position and that also extends in a substantially horizontal direction when the holding arm 20 is arranged in the loading position, and that furthermore extends substantially horizontal when the holding arm 20 is arranged in any position between the storage position and the loading position.

The first connecting device 30 and the second connecting device 60 are coupled to each other by means of a belt 70 that is designed as a toothed belt and that is connected to the first connecting device 30 and to the second connecting device 60. With the belt 70 it can be achieved that the first connecting device 30 and the second connecting device 60 are coupled so that a rotation of the holding arm 20 about the holding arm axis 200 then inevitably causes a rotation of the receiving element 40 about the receiving element axis 400, and vice versa. This way, the first angle of rotation R1 and the second angle of rotation R2 are equal in amount in any position of the holding arm 20.

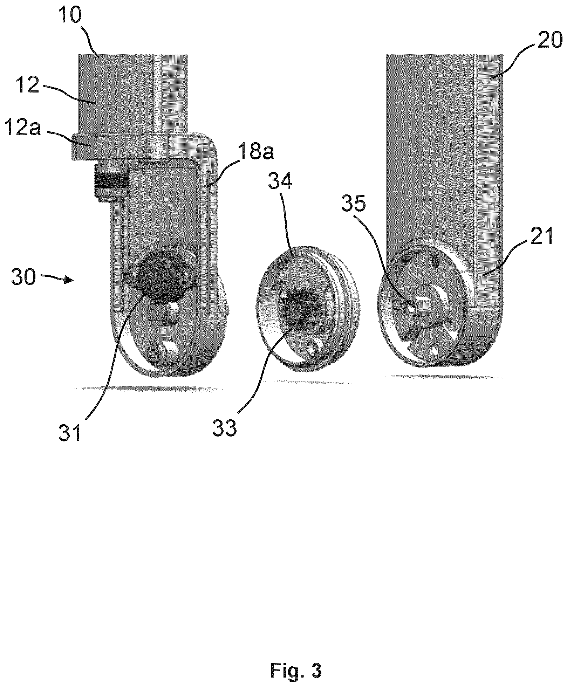

Fig. 3 shows an exploded view of the connection of the first end 21 of the holding arm 20 with the second end 12 of the fixed element 10 by means of a first connecting device 30. The first end 21 of the holding arm 20 is rotatably connected to the second end 12 of the fixed element 10 via the rotary damper holder 12a that can be considered to be a part of the second end 12 of the fixed element 10. The rotary damper holder 12a comprises a slit 18a behind which a light-emitting diode can be arranged for indicating a state of the holding device and/or a state of a medical element that is arranged on the receiving element of the holding device. In this embodiment, the rotary damper 30a comprises one damping gear 31 that meshes with a first connecting device gear 33 that is arranged in an intermediate element 34 that can be connected to a connecting element 35 of the first end 21 of the holding arm.

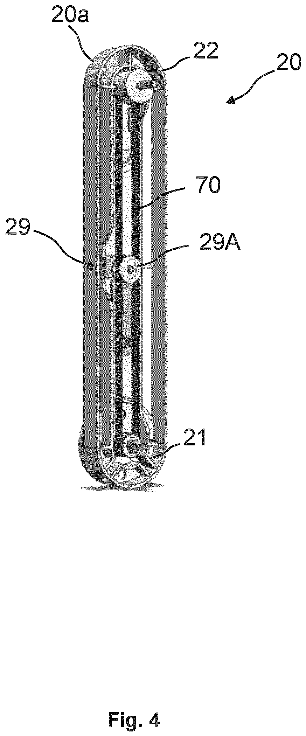

Fig. 4 shows a perspective view of the inner part of the holding arm 20. The holding arm 20 comprises a housing 20a (one side of the housing is not shown here). The belt 70 is enclosed by the housing and arranged inside the holding arm 20. A tensioning wheel 29A is in contact with the belt 70 and configured and arranged to tension the belt 70. The tensioning wheel 29A is movable relative to the belt 70 for tensioning the belt 70. The tensioning wheel 29A can be moved by using a screw driver. A screw driver can be inserted through the hole 29 that is arranged in the housing 20a.

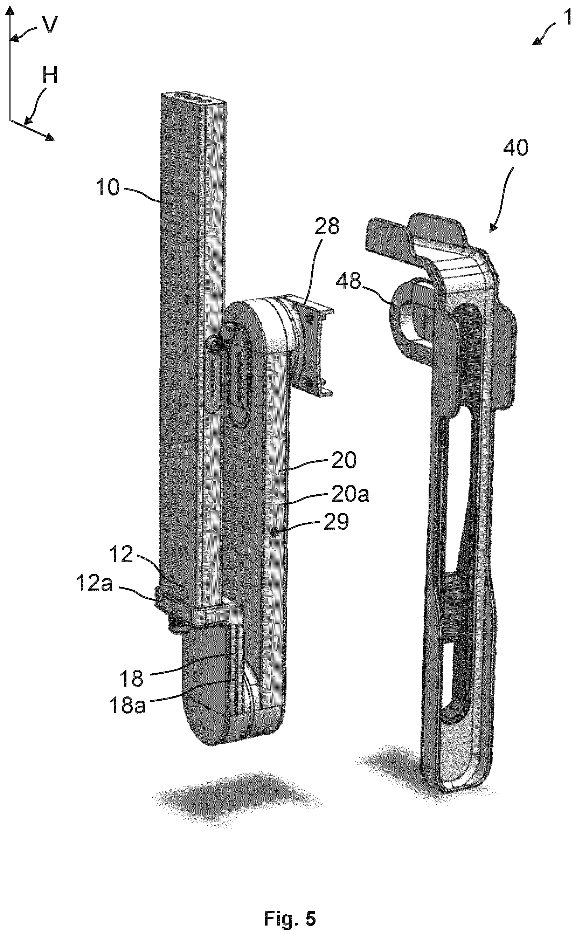

Fig. 5 shows a perspective view of a holding device 1, wherein the holding arm 20 is arranged in the storage position and the receiving element 40 is removed. The receiving element 40 is detachably connectable to the holding arm 20 by a connection between the holding arm connecting part 28 and the receiving element connecting part 48. This way, different receiving elements that have a receiving element connecting part 48 as shown here can be connected to the holding arm connecting part 28.

The holding device 1 comprises a state indicator device 18 that is configured to indicate a state of the holding device 1 and/or a state of a medical instrument that is arranged on the receiving element 40. The state indicator device 18 is arranged at the fixed element 10. The state indicator device 18 can show a status of the device and/or of the medical instrument by displaying a state. The state indicator device 18 is integrated in the fixed element 10 at the second end 12 of the fixed element, in particular at the rotary damper holder 12a. The state indicator device 18 comprises at least one light-emitting diode (not shown) that is arranged behind a slit 18a. The state indicator device 18 can display different states of the holding device and/or of a medical instrument dependent on sensor data received. For example the state indicator device 18 can display the following states as follows: 1. the holding device is free to use and/or no medical instrument is arranged on the receiving element is displayed by a white light; 2. a medical instrument that is arranged on the receiving element is being dried is displayed by a blue light; 3. a medical instrument that is arranged on the receiving element is dry is displayed by a green light; 4. a problem is detected is displayed by a red light.

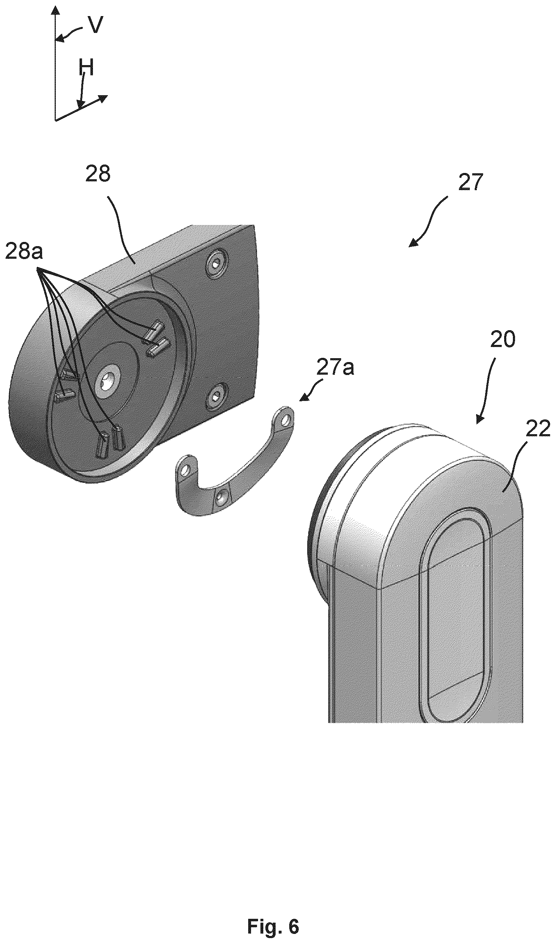

Fig. 6 shows an exploded view of the engaging mechanism 27. The engaging mechanism 27 comprises a spring element 27a that is formed as a spring plate. The holding arm connecting part 28 comprises latching elements 28a. The engaging mechanism 27 is configured to provide a locking force to the holding arm 20 and therefore also to the receiving element when the holding arm 20 is arranged in the storage position. The engaging mechanism 27 is further configured to provide a locking force to the holding arm 20 and therefore also to the receiving element when the holding arm 20 is arranged in the loading position.

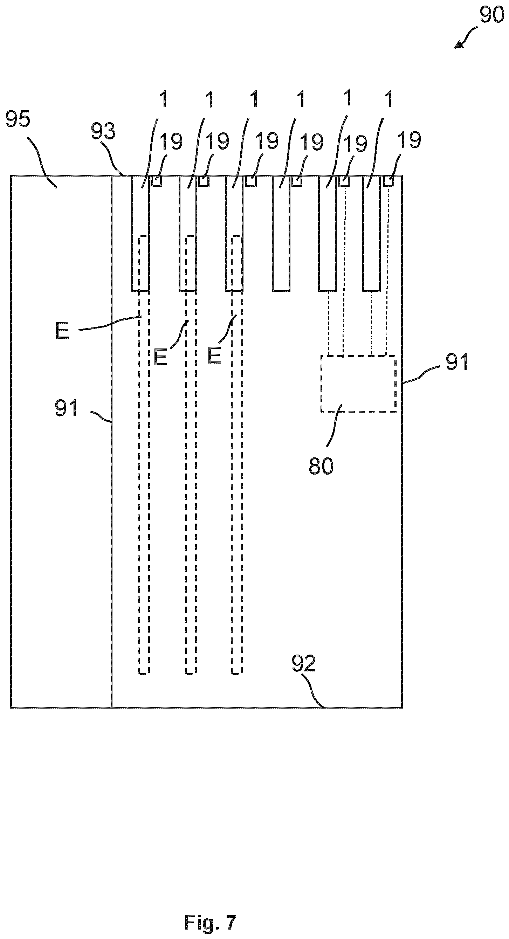

Fig. 7 shows a schematic view of a cabinet 90 for storing and/or drying a medical instrument, namely an endoscope E. The cabinet 90 comprises side walls 91, a bottom wall 92, a ceiling element 93 and a closable door 95 (here shown in an opened position). In the cabinet 90 six holding devices 1 are arranged at the upper part of the cabinet 90. The holding devices 1 are connected to the ceiling element 93. The holding devices 1 are configured to hold endoscopes E (only three endoscopes are schematically shown here, the holding devices 1 on the right can also hold endoscopes). Next to each holding device 1 a sensor 19 for obtaining sensor data is arranged on the ceiling element 93. The sensors 19 can detect if an endoscope is placed on the respective holding device 1. The sensor can further detect if the endoscope is being dried and/or if the endoscope has been dried. The sensor can further detect errors. Dependent on the sensor data obtained by the sensors 19, the state indicator devices (not shown in this figure) of the holding devices can display a corresponding state by displaying a light in a color that is assigned to the specific state. A control unit 80 can optionally be provided and signally connected to the sensors 19 and to the state indicator device. Such a control unit 80 can process the sensor data obtained and dependent on these sensor data provide instruction signals to the state indicator devices to display a light in a color that is assigned to the specific state that has been determined.

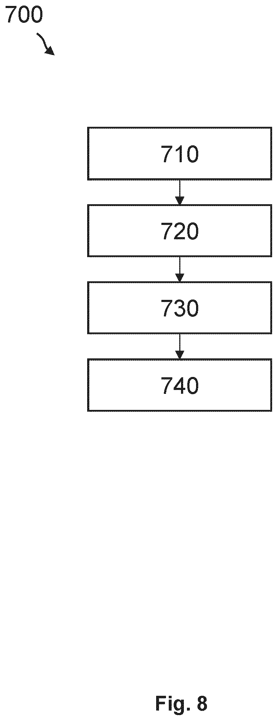

Fig. 8 shows a schematic view of a method 700 for holding a medical instrument. The method comprises the following steps: In a step 710, providing a holding device 1, preferably a holding device as described herein, wherein the holding device 1 comprises: a fixed element 10, a holding arm 20, wherein the holding arm 20 is rotatably connected to the fixed element 10 in such a way that the holding arm 20 is rotatable about a holding arm axis 200 relative to the fixed element 10, and a receiving element 40 for receiving a medical instrument, in particular an endoscope, wherein the receiving element 40 is rotatably connected to the holding arm 20 in such a way that the receiving element 40 is rotatable about a receiving element axis 400 relative to the holding arm 20. In a step 720, moving the holding arm 20 from a storage position, in which the holding arm 20 is arranged substantially vertically, to a loading position by rotating the holding arm 20 about the holding arm axis 200 through a first angle of rotation R1, whereby the receiving element 40 is rotated about the receiving element axis 400 through a second angle of rotation R2, wherein preferably the first angle of rotation R1 and the second angle of rotation R2 are substantially equal in amount. In a step 730, placing a medical instrument, in particular an endoscope, onto the receiving element 40. In a step 740, rotating the holding arm 20 about the holding arm axis 200 back into the storage position.

### List of Reference Signs

- 1: holding device
- 10: fixed element
- 11: first end of the fixed element
- 12: second end of the fixed element
- 12a: rotary damper holder of the fixed element
- 15: longitudinal extension of the fixed element
- 18: state indicator device
- 18a: slit
- 19: sensor
- 20: holding arm
- 200: holding arm axis
- 20a: housing of the holding arm
- 21: first end of the holding arm
- 22: second end of the holding arm
- 25: longitudinal extension of the holding arm
- 27: engaging mechanism
- 27a: spring element
- 28: holding arm connecting part
- 28a: latching elements
- 29: hole
- 29A: tensioning wheel
- 30: first connecting device
- 30a: rotary damper
- 31, 32: rotary damping gears
- 33: first connecting device gear
- 34: intermediate element
- 35: connecting element
- 40: receiving element
- 400: receiving element axis
- 41: first support part of the receiving element
- 41a: first support surface of the receiving element
- 42: second support part of the receiving element
- 42a: second support surface of the receiving element
- 45: longitudinal extension of the receiving element
- 48: receiving element connecting part
- 60: second connecting device
- 70: belt
- 80: control unit
- 90: cabinet
- 91: side walls of the cabinet
- 92: bottom wall of the cabinet
- 93: ceiling element of the cabinet
- 95: closable door of the cabinet
- 700: method
- 710-740: method steps
- E: endoscope
- H: horizontal direction
- V: vertical direction
- R1: first angle of rotation
- R2: second angle of rotation

## Claims

1. A holding device (1) for holding a medical instrument, in particular an endoscope, the holding device (1) comprising:
- a fixed element (10) that extends from a first end (11) of the fixed element (10) to a second end (12) of the fixed element (10),
- a holding arm (20) that extends from a first end (21) of the holding arm (20) to a second end (22) of the holding arm (20), wherein the first end (21) of the holding arm (20) is rotatably connected to the second end (12) of the fixed element (10) in such a way that the holding arm (20) is rotatable about a holding arm axis (200) relative to the fixed element (10) and movable by rotating the holding arm (20) about the holding arm axis (200) between a storage position and a loading position, and
- a receiving element (40) for receiving a medical instrument, in particular an endoscope, wherein the receiving element (40) is rotatably connected to the second end (22) of the holding arm (20) in such a way that the receiving element (40) is rotatable about a receiving element axis (400) relative to the holding arm (20).

2. The holding device according to the preceding claim,
wherein the holding arm (20) has a longitudinal extension (25) between the first end (21) of the holding arm (20) and the second end (22) of the holding arm (20), and wherein the holding arm (20) is arranged in such a way that
- in the storage position the longitudinal extension (25) of the holding arm (20) is substantially vertical and the second end (22) of the holding arm (20) lies vertically above the first end (21) of the holding arm (20), and/or
- in the loading position the longitudinal extension of the holding arm is not substantially vertical,
wherein preferably in the storage position the second end (22) of the holding arm (20) is arranged in an upper position and in the loading position the second end (22) of the holding arm (20) is arranged in a lower position.

3. The holding device according to at least one of the preceding claims, wherein the fixed element (10) has a longitudinal extension (15) between the first end (11) of the fixed element (10) and the second end (12) of the fixed element (10), wherein the longitudinal extension (15) of the fixed element (10) is substantially vertical and the first end (11) of the fixed element (10) lies vertically above the second end (12) of the fixed element (10).

4. The holding device according to at least one of the preceding claims, wherein the receiving element (40) comprises a first support part (41) with a first support surface (41a) that has a longitudinal extension (45) of the receiving element (40), wherein the longitudinal extension (45) of the receiving element (40) is substantially vertical when the holding arm (20) is arranged in the storage position and substantially vertical when the holding arm (20) is arranged in the loading position, and preferably substantially vertical when the holding arm (20) is arranged in any position between the storage position and the loading position, and/or wherein the receiving element (40) comprises a second support part (42) with a second support surface (42a) that extends in a substantially horizontal direction when the holding arm (20) is arranged in the storage position and that extends in a substantially horizontal direction when the holding arm (20) is arranged in the loading position, and that preferably extends substantially horizontal when the holding arm (20) is arranged in any position between the storage position and the loading position.

5. The holding device according to at least one of the preceding claims,
wherein the holding arm axis (200) and the receiving element axis (400) are arranged parallel to each other and preferably horizontally, and/or
wherein in the loading position of the holding element (20) the holding arm axis (200) is arranged in a vertically higher position than the receiving element axis (400), and/or
wherein in the storage position of the holding element (20) the holding arm axis (200) is arranged in a vertically lower position than the receiving element axis (400), and/or
wherein the holding arm (20) is rotatable about the holding arm axis (200) through a first angle of rotation (R1) between the storage position and the loading position, wherein the receiving element (40) is rotatable about the receiving element axis (400) through a second angle of rotation (R2) between the storage position and the loading position,
and wherein the first angle of rotation (R1) and the second angle of rotation (R2) are substantially equal in amount,
wherein preferably the first angle of rotation (R1) and the second angle of rotation (R2) are each larger than 90°.

6. The holding device according to at least one of the preceding claims,
wherein the first end (21) of the holding arm (20) is rotatably connected to the second end (12) of the fixed element (10) by means of a first connecting device (30), and wherein the receiving element (40) is rotatably connected to the second end (22) of the holding arm (20) by means of a second connecting device (60), and/or
wherein the first connecting device (30) is configured and arranged to rotate the holding arm (20) about the holding arm axis (200) through a first angle of rotation (R1) between the storage position and the loading position,
wherein the second connecting device (60) is configured and arranged to rotate the receiving element (40) about the receiving element axis (400) through a second angle of rotation (R2) between the storage position and the loading position,
and wherein the first angle of rotation (R1) and the second angle of rotation (R2) are substantially equal in amount.

7. The holding device according to at least one of the preceding claims,
wherein the first connecting device (30) and the second connecting device (60) are coupled to each other in such a way that a rotation of the holding arm (20) about the holding arm axis (200) causes a rotation of the receiving element (40) about the receiving element axis (400) that is substantially equal in amount, and/or
wherein the first connecting device (30) and the second connecting device (60) are coupled to each other by means of a belt (70), in particular a toothed belt (70), that is connected to the first connecting device (30) and to the second connecting device (60),
wherein preferably the belt is enclosed by a housing (20a) of the holding arm (20).

8. The holding device according to at least one of the preceding claims, wherein the holding arm (20) comprises a tensioning wheel (29A) that is in contact with the belt (70) and configured and arranged to tension the belt (70).

9. The holding device according to at least one of the preceding claims,
wherein the first connecting device (30) comprises a rotary damper (30a) that is connected to the second end (12) of the fixed element (10), in particular via a rotary damper holder (12a) that is connected to the second end (12) of the fixed element (10), wherein the rotary damper (30a) comprises at least one rotary damping gear (31, 32), preferably two rotary damping gears (31, 32),
wherein preferably the at least one rotary damping gear (31, 32) meshes with a first connecting device gear (33) that is connected to the first end (21) of the holding arm (20) in a rotationally fixed manner,
and/or wherein preferably the at least one damping gear (31, 32) is configured and arranged to provide a damping torque against a rotational movement of the holding arm (20) about the holding arm axis (200) for slowing down a rotation of the holding arm (20) about the holding arm axis (200).

10. The holding device according to at least one of the preceding claims, wherein the holding device comprises an engaging mechanism (27), preferably comprising a spring element (27a), in particular formed as a spring plate, and/or at least one latching element (28a), wherein the engaging mechanism (27) is configured and arranged to provide a locking force to the holding arm (20) and/or to the receiving element when the holding arm (20) is arranged in the storage position and/or to provide a locking force to the holding arm (20) and/or to the receiving element when the holding arm (20) is arranged in the loading position.

11. The holding device according to at least one of the preceding claims,
wherein the holding device (1) comprises a state indicator device (18) that is configured to indicate a state of the holding device (1) and/or a state of a medical instrument that is arranged on the receiving element (40), wherein preferably the state indicator device (18) is arranged on the fixed element (10),
wherein preferably the state indicator device (18) is configured to display different states of the holding device and/or of a medical instrument that is arranged on the receiving element (40) by means of at least one state light that is configured to show different colors for different states of the holding device and/or of a medical instrument that is arranged on the receiving element (40), preferably dependent on sensor data that is obtained by at least one sensor and/or dependent on the duration of a medical instrument being arranged on the receiving element (40), wherein preferably the different states comprise at least one, in particular all, of the following states:
- the holding device is free to use and/or no medical instrument is arranged on the receiving element;
- a medical instrument that is arranged on the receiving element is being dried;
- a medical instrument that is arranged on the receiving element is dry;
- a problem is detected.

12. A cabinet (90) for storing and/or drying a medical instrument, in particular an endoscope, the cabinet (90) comprising:
- at least one holding device (1), preferably several holding devices (1), according to at least one of the preceding claims,
wherein the at least one holding element (1) is arranged inside of the cabinet (90),
wherein preferably the fixed element (10) of the at least one holding device (1) is mounted to a ceiling element (93) of the cabinet (90),
wherein further preferably at least one sensor (19) for obtaining sensor data is arranged in the upper part of the cabinet (90), in particular on the inner side of the ceiling element (93) of the cabinet (90).

13. A method (700) for holding a medical instrument, in particular an endoscope, the method comprising:
- providing (710) a holding device (1), preferably a holding device according to at least one of the claims 1-11, wherein the holding device (1) comprises:
∘ a fixed element (10),
∘ a holding arm (20), wherein the holding arm (20) is rotatably connected to the fixed element (10) in such a way that the holding arm (20) is rotatable about a holding arm axis (200) relative to the fixed element (10), and
∘ a receiving element (40) for receiving a medical instrument, in particular an endoscope, wherein the receiving element (40) is rotatably connected to the holding arm (20) in such a way that the receiving element (40) is rotatable about a receiving element axis (400) relative to the holding arm (20),
- moving (720) the holding arm (20) from a storage position, in which the holding arm (20) is arranged substantially vertically, to a loading position by rotating the holding arm (20) about the holding arm axis (200) through a first angle of rotation (R1), whereby the receiving element (40) is rotated about the receiving element axis (400) through a second angle of rotation (R2), wherein preferably the first angle of rotation (R1) and the second angle of rotation (R2) are substantially equal in amount.

14. The method according to the preceding claim, comprising:
- placing (730) a medical instrument, in particular an endoscope, onto the receiving element (40), and
- rotating (740) the holding arm (20) about the holding arm axis (200) back into the storage position, wherein preferably when the storage position and/or the loading position is reached a haptic and/or acoustic feedback is generated by an engaging mechanism (27), preferably comprising a spring element (27a) and/or at least one latching element (18a).

15. A use of a holding device (1) according to at least one of the claims 1-11 for holding an endoscope, in particular in a cabinet (90) according to claim 12.
